# EUROPEAN PATENT APPLICATION

(11) **EP 2 223 704 A1**
(43) Date of publication of application: **01.09.2010**
(21) Application number: 09002200.5
(22) Date of filing: 17.02.2009
(51) Int. Cl.: A61L 2/14, H05H 1/00

(54) **Treating device for treating a body part of a patient with a non-thermal plasma**

(71) Applicant: Max-Planck-Gesellschaft zur Förderung der Wissenschaften e.V., 80539 München (DE)
(72) Inventor: The designation of the inventor has not yet been filed
(74) Representative: Beier, Ralph

(57) **Abstract**

The invention relates to a treating device (1) for treating a body part of a patient with a non-thermal plasma, particularly for sterilizing a hand of a human being, said treating device (1) comprising a housing (2) for temporarily receiving the body part within the housing (2) during the treatment and for applying the plasma to the body part within the housing (2), and an inlet opening (3) being arranged in the housing (2) for introducing the body part through the inlet opening (3) into the housing (2).
The device might further comprise a plasma generator (6,7) with two electrodes (9,10) and a barrier (8) between the electrodes; a high voltage generator; an outer electric insulation (11); gas permeable radiation shielding (12) and a spacer.

## Description

### Field of the invention

The invention relates to a treating device for treating a body part of a patient with a non-thermal plasma, particularly for sterilizing a hand of a human being.

### Background of the invention

The use of non-thermal plasma for the treatment of wounds and especially for the in-vivo sterilization, decontamination or disinfection of wounds is disclosed, for example, in WO 2007/031250 A1 and PCT/EP2008/003568. However, the known devices for plasma treatment are suitable to only a limited extent for the in-vivo sterilization of a hand of a human being.

### Summary of the invention

Therefore, it is a general object of the invention to provide a treating device which is suitable for the in-vivo sterilization of a hand of a human being.

This object is achieved by a novel treating device according to the main claim.

The treating device according to the invention comprises a housing for temporarily receiving a body part which is to be sterilized within the housing during the treatment and for applying the non-thermal plasma to the body part within the housing. Therefore, the treating device according to the invention is different in nature from conventional treating devices in which the object of the treatment (e.g. a hand) is located outside a plasma applicator so that the plasma applicator must be moved along the surface of the object of treatment so that the non-thermal plasma is applied to the entire surface of the object of treatment. In other words, in conventional treating devices the non-thermal plasma is applied to the object of treatment while the invention provides that the object of treatment (e.g. a hand of a human being) is introduced into the non-thermal plasma so that the object of treatment is completely surrounded by the non-thermal plasma.

The housing of the treating device according to the invention comprises an inlet opening for introducing the body part (e.g. a hand of a human being) through the inlet opening into the housing so that the plasma treatment takes place within the housing.

The treating device according to the invention is particularly suitable for the in-vivo sterilization of a hand of a human being. However, the treating device according to the invention can also be used for the plasma treatment of other body parts of a patient, e.g. a foot. Furthermore, the object of treatment can be a non-biological article like a surgical instrument.

Further, the treating device according to the invention preferably comprises an integrated plasma generator for generating the non-thermal plasma within the housing. Therefore, the plasma generator is preferable an integral part of the treating device.

Alternatively, it is possible that the treating device merely comprises an inlet for introducing the plasma into the housing wherein the plasma is generated outside the housing by a separate plasma generator which can be connected with the inlet of the treating device via a hose.

In a preferred embodiment of the invention, the plasma generator comprises at least two electrodes and a barrier between the electrodes, so that the plasma is generated between the electrodes by a dielectric barrier discharge (DBD) which is per se known in the state of the art. Therefore, the barrier between the electrodes preferably consists of an electrically insulating and/or dielectric material, particularly polytetraflouroethylene.

Further, the electrodes can be adhered to the barrier on opposite sides of the barrier.

Moreover, the electrode(s) is/are preferably connected with a high voltage generator which can be arranged separate from the treating device.

The housing of the treating device according to the invention is preferably box-shaped, whereas there are two of the afore mentioned sandwich-like DBD arrangements within the housing above and below the area of treatment. Alternatively, the DBD arrangements can be mounted on opposing sides of the housing so that one DBD arrangement is mounted on the left side of the housing, whereas the other DBD arrangement is mounted on the right side of the housing.

Further, the afore mentioned sandwich-like DBD arrangement preferably comprises an outer electric insulation which is electrically insulating the outer electrode of the plasma generator.

Moreover, there is preferably a gap between the outer electric insulation of the sandwich-like DBD arrangement and the housing, wherein said gap allows a gas flow through the gap. This is advantageous since the plasma generated in the DBD arrangement must reach the area of treatment in the centre of the housing so that there must be a gas flow within the housing. The gas flow within the housing can be generated by natural convection due to the different temperatures within the gas volume. However, it is also possible that the gas circulation within the housing of the treating device is at least partially caused by a pump which is preferably arranged separate from the treating device.

It should further be mentioned that the plasma generator is preferably arranged within the housing so that the plasma is generated within the housing. Therefore, the treating device according to the invention is different in nature from conventional therapeutic concepts in which the area of treatment and the area of plasma generation are separated from each other. On the contrary, the invention provides that the area of treatment and the area of plasma generation are at least overlapping or even identical.

It is well known in the state of the art that plasma generators generally produce ultraviolet (UV) radiation. In some applications this UV radiation contributes to the therapeutic effect of the plasma treatment. However, in other applications, the UV radiation is undesirable. Therefore, the treating device according to the invention preferably comprises a radiation shielding being arranged between the plasma generator and the area of treatment within the housing thereby shielding the treated body part against the UV radiation generated by the plasma generator.

However, the afore mentioned radiation shielding is preferably gas permeable so that the plasma can flow through the radiation shielding and reach the body part which is to be treated. This is important since the plasma treatment requires a physical contact between the non-thermal plasma and the body part which is to be treated.

In a preferred embodiment, the radiation shielding comprises several spaced apart UV blocking shielding elements which are curved or angled in such a way that there is no intervisibility between the opposing sides of the radiation shielding while the gas flow between the opposing sides of the radiation shielding is not substantially constricted.

The shielding elements are preferably lamellas which are arranged in at least two adjacent layers wherein the lamellas in the adjacent layers are oppositionally angled.

It should further be mentioned that the radiation shielding and/or the shielding elements (e.g. lamellas) preferably consist of or a coated with an electrically conductive material so that there is not charge build-up on the surface of the shielding elements. The electrically conductive material of the radiation shielding is preferably metal, particularly copper or tin. It should further be mentioned that the radiation shielding and/or the shielding elements are preferably electrically grounded.

In the preferred embodiment of the invention, the electrodes, the barrier and the outer insulation of the afore mentioned DBD arrangement are preferably flat or layer-shaped. Further, the electrodes can comprise a wire mesh.

Further, the treating device preferably comprises a spacer which is arranged between the area of treatment on the one hand and the plasma generator on the other hand thereby preventing a physical contact between the plasma generator and the body part during treatment. The spacer is preferably substantially flat and/or comprises a wire mesh.

Moreover, it should be noted that the housing of the novel treating device preferably comprises an outer wall consisting of an electrically conductive material which is preferably electrically grounded.

The dimensions of the housing are preferably adapted to the size of a hand of a human being so that a patient can introduce his hand through the inlet opening into the housing for sterilizing his hand. Therefore, the inlet opening of the housing preferably comprises a height in the range of 2cm-20cm and a width in the range of 5cm-30cm. It is preferred that the inlet opening of the housing comprises a width of 10cm and a height of 4cm.

Further, the housing is preferably sufficiently large for introducing a hand of a human being into the housing so that the entire hand can be sterilized within the housing. Therefore, the housing preferably comprises an inner length in the range of 5cm-30cm with a preferred value of the inner length of about 11-12cm. Further, the housing preferably comprises an inner width in the range of 5cm-30cm with a preferred value of the width of about 11-12cm. Finally, the housing preferably comprises an inner height in the range of 4cm-20cm with a preferred value of the inner height of about 7cm.

It should further be noted that the non-thermal plasma according to the invention preferably comprises a gas temperature (i.e. the temperature of the atoms and molecules) below +40°C, when measured on the treated surface.

Further, the treating device can include an on/off-switch for switching the integrated plasma generator on and off.

Moreover, there can be a light barrier which detects whether an object of treatment (e.g. a hand) is inserted through the inlet opening into the housing. The light barrier can be coupled with the plasma generator so that the plasma generator is switched off if no object is introduced through the inlet opening, whereas the plasma generator is switched on if an object of treatment is present within the housing.

The invention and its particular features and advantages will become apparent from the following detailed description considered with reference to the accompanying drawings.

### Brief description of the drawings

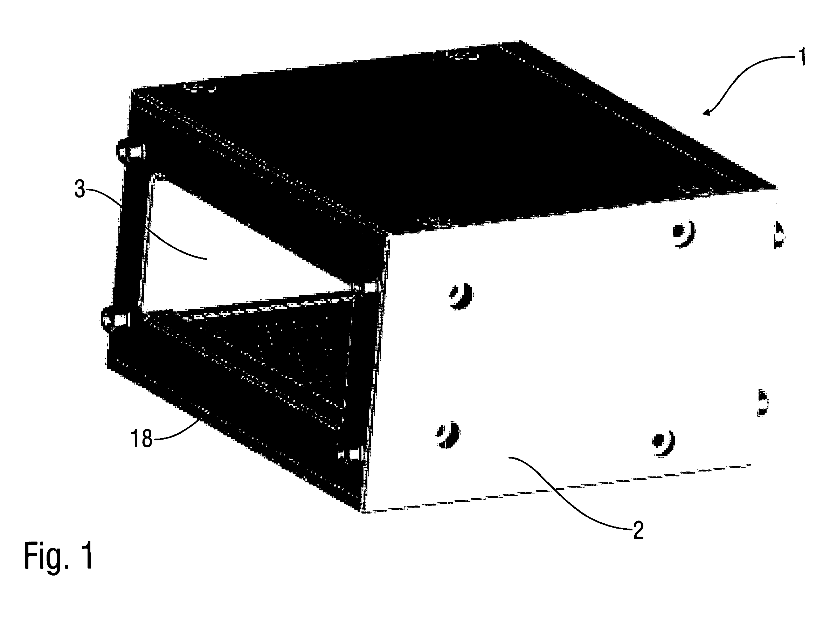

- Figure 1: shows a perspective view of a preferred embodiment of a treating device according to the invention.

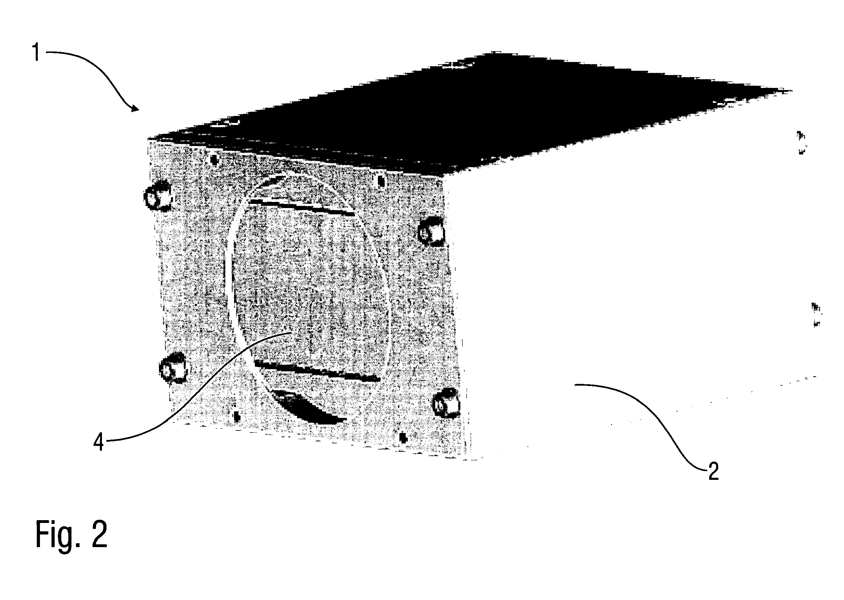

- Figure 2: shows another perspective view of the treating device according to Figure 1.
- Figure 3: shows a cross sectional view of the treating device shown in Figures 1 and 2.
- Figure 4: shows a schematic view of a plasma generator using dielectric barrier discharge.
- Figure 5: shows a cross sectional view of the radiation shielding shown in Figure 4.

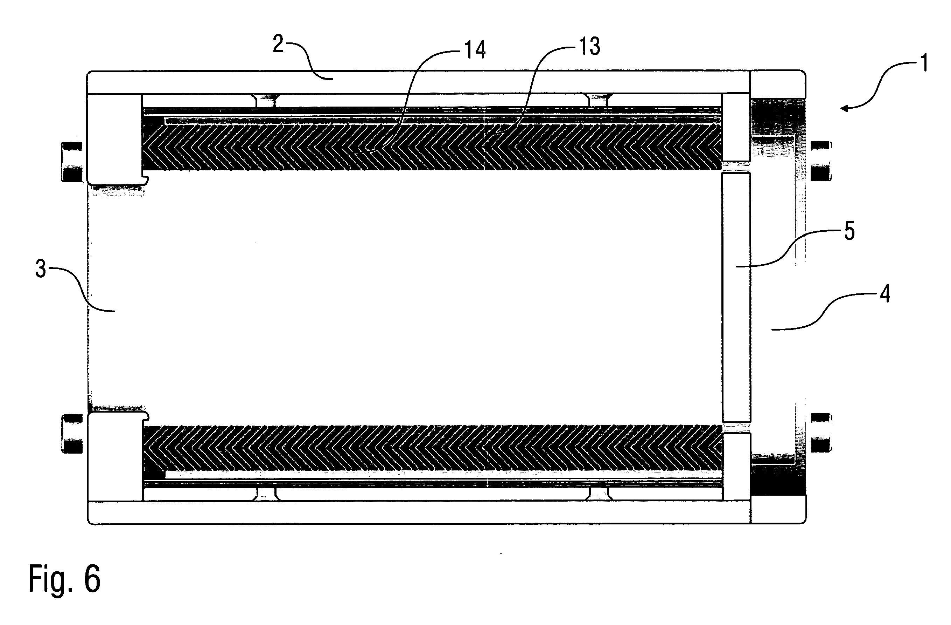

- Figure 6: shows a cross sectional view similar to Figure 3 but also showing the design of the radiation shielding.
- Figure 7A: shows a perspective view of a side plate of the housing of the treating device.
- Figure 7B: shows a perspective view of the isolator of the DBD arrangement.
- Figure 7C: shows a perspective view of the front plate of the treating device with an inlet opening.
- Figure 7D: shows a perspective view of an intermediate plate of the treating device.
- Figure 7E: shows a perspective view of a rear plate of the treating device comprising an opening for cables.
- Figure 7F: shows a perspective view of an upper and lower plate of the housing.
- Figure 7G: shows an exemplary embodiment of the electrodes of the afore mentioned DBD arrangement.
- Figure 8: shows another embodiment of an electrode arrangement which can be used for plasma generation instead of the DBD arrangement.

### Detailed description of the drawings

The drawings illustrate a preferred embodiment of a treating device 1 for the in-vivo sterilization of a hand of a human being by means of a non-thermal plasma.

The treating device 1 comprises a box-shaped housing 2 with an inlet opening 3 at the front side of the housing 2 wherein the dimensions of the inlet opening 3 are adapted to the size of a hand of a human being so that a patient can introduce his hand through the inlet opening 3 into the housing 2 of the treating device 1. Further, the dimensions of the entire housing 2 are adapted to the size of a hand of a human being so that the entire hand can be placed within the housing 2 for a plasma treatment. In this embodiment, the housing 2 comprises a length of 11,5cm, a width of 11,4cm and a height of 7cm. Further, the inlet opening 3 comprises a width of 10cm and a height of 4cm.

Further, the treating device 1 comprises an opening 4 at its rear surface opposite the inlet opening 3 while the opening 4 serves for accommodating cables or the like. However, the rear opening 4 is covered by an insulator 5 consisting of polytetraflouroethylene.

Further, the treating device 1 comprises an integrated plasma generator which generates a non-thermal plasma for the in-vivo sterilization.

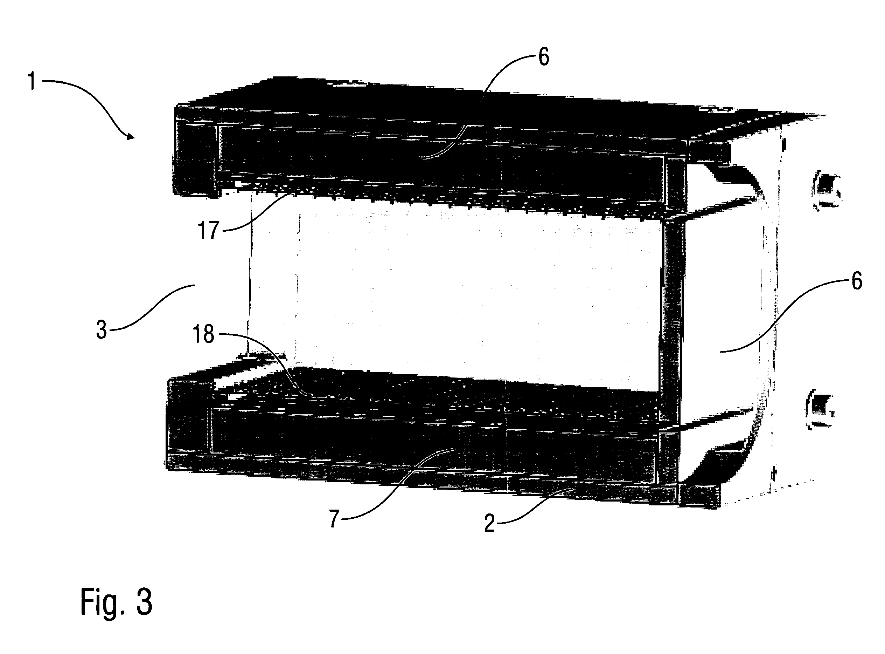

The plasma generator comprises two substantially flat dielectric barrier discharge (DBD) arrangements 6, 7. The DBD arrangement 6 is arranged within the housing 2 above the area of treatment as shown in Figure 3, while the DBD arrangement 7 is arranged within the housing 2 below the area of treatment.

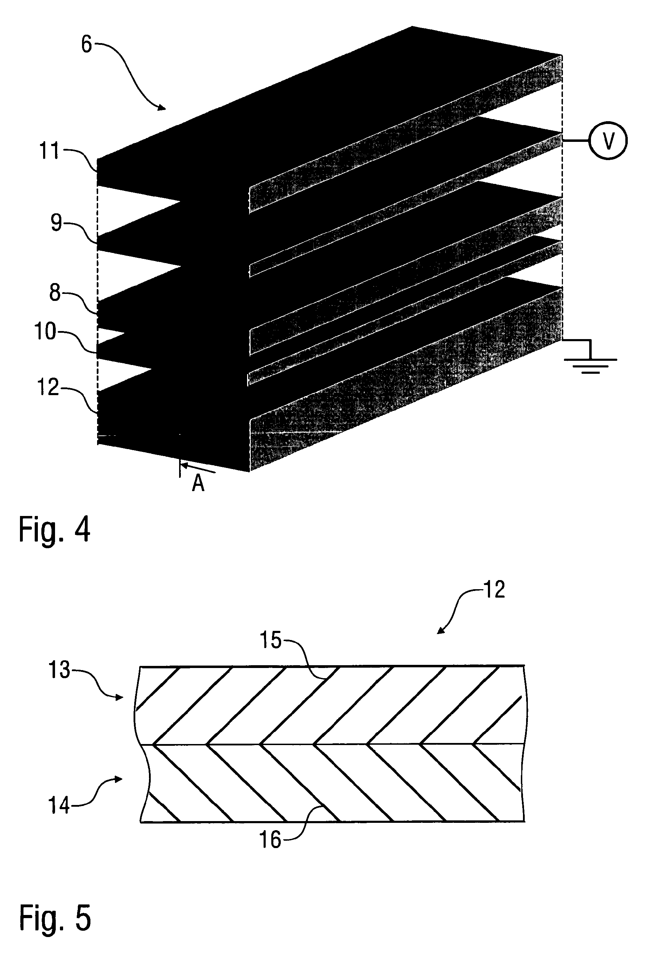

The design of the DBD arrangements 6, 7 is schematically shown in Figure 4. Each of the DBD arrangements 6, 7 comprises a barrier 8 sandwiched between two electrodes 9, 10 which are adhered to the top and bottom sides of the barrier 8 which consists of polytetraflouroethylene.

Further, the DBD arrangement 6 comprises an outer insulator 11 and a radiation shielding 12 facing to the area of treatment within the housing 2 so that the radiation shielding 12 prevents that the hand of the patient within the housing 2 is affected by any ultraviolet radiation generated by the DBD arrangements 6, 7.

Figure 5 shows a cross sectional view of the radiation shielding 12 along line A-A in Figure 4. The radiation shielding 12 comprises two adjacent layers 13, 14 of parallel metallic lamellas 15, 16. The lamellas 15 in the upper layer 13 of the radiation shielding 12 are oppositionally angled with regard to the lamellas 16 in the lower layer 14 of the radiation shielding 12. Therefore, there is no intervisibility between the opposing sides of the radiation shielding 12 so that no ultraviolet radiation is transmitted through the radiation shielding 12. In other words, the radiation shielding 12 blocks any ultraviolet radiation generated by the DBD arrangements 6, 7.

Further, the treating device 1 comprises two spacers 17, 18 for the DBD arrangements 6, 7, wherein the spacers 17, 18 avoid a physical contact between the hand and the DBD arrangements 6, 8. In this embodiment, the spacers 17, 18 each consist of a wire mesh.

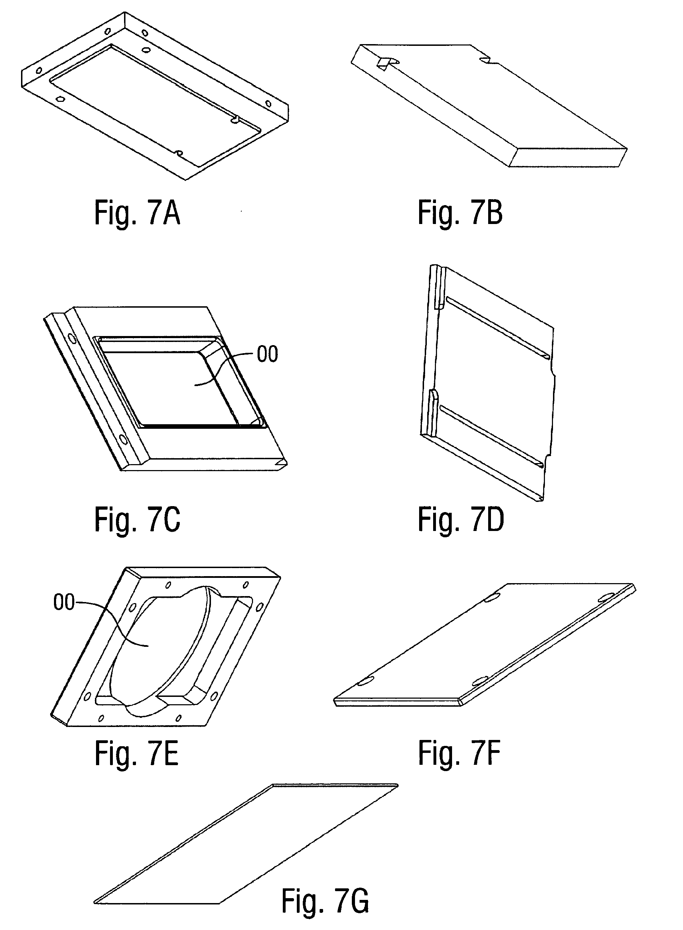

Figures 7A-7G show different views of the parts of the afore mentioned treating device while the views are self explanatory so that no further explanation is necessary.

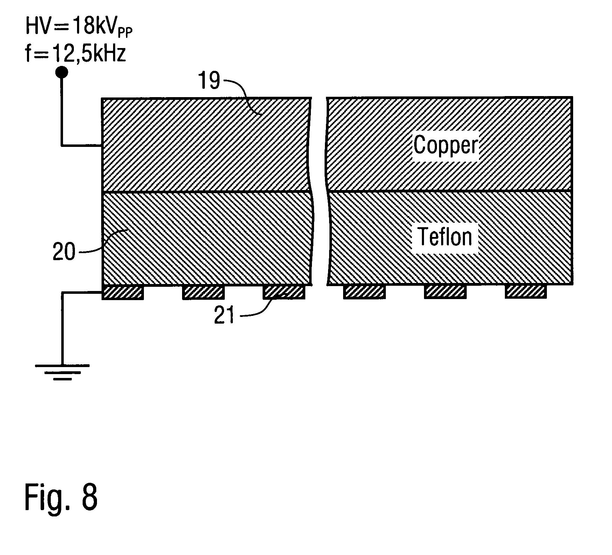

Figure 8 shows another embodiment of an electrode arrangement which can be used instead of the afore-mentioned DBD arrangements 6, 7.

The electrode arrangement comprises a copper plate 19, a teflon plate 20 and a wire mesh 21 made of an electrically on-cudctive material. The copper plate 19 and the wire-mesh 21 are adhered to opposing sides of the teflon plate 20.

Further, the wire mesh 21 is electrically grounded, whereas the copper plate 19 is connected with a high voltage source generating a high-voltage of U=18kV_{PP} and a frequency of f=12,5kHz.

Although the invention has been described with reference to the particular arrangement of parts, features and the like, these are not intended to exhaust all possible arrangements of features, and indeed many other modifications and variations will be ascertainable to those of skill in the art.

### List of reference numerals:

- 1: Therapeutic device
- 2: Housing
- 3: Inlet opening
- 4: Opening
- 5: Insulator
- 6: DBD arrangement
- 7: DBD arrangement
- 8: Barrier
- 9: Electrode
- 10: Electrode
- 11: Outer insulation
- 12: Radiation shielding
- 13: Layer
- 14: Layer
- 15: Lamellas
- 16: Lamellas
- 17: Spacer
- 18: Spacer
- 19: Copper plate
- 20: Teflon plate
- 21: Wire mesh

## Claims

1. Treating device (1) for treating an object with a non-thermal plasma, particularly for the in-vivo sterilization of a hand of a human being,
**characterized by**
a) a housing (2) for temporarily receiving the object within the housing (2) during the treatment and for applying the plasma to the object within the housing (2), and
b) an inlet opening (3) being arranged in the housing (2) for introducing the object through the inlet opening (3) into the housing (2).

2. Treating device (1) according to claim 1, further comprising
a) an integrated plasma generator (6, 7) for generating the non-thermal plasma within the housing (2), or
b) an inlet for introducing the plasma into the housing (2) wherein the plasma is generated outside the housing (2).

3. Treating device (1) according to claim 2, wherein
a) the plasma generator (6, 7) comprises at least two electrodes (9, 10) and a barrier (8) between the electrodes (9, 10), so that the plasma is generated between the electrodes (9, 10) by a dielectric barrier discharge, and/or
b) the barrier (8) between the electrodes consists of an electrically insulating and/or dielectric material, particularly polytetrafluoroethylene, and/or
c) the electrode (9, 10) is adhered to the barrier (8), and/or
d) at least one of the electrodes (9, 10) is connected with a high voltage generator.

4. Treating device (1) according to claim 3, further comprising:
a) an outer electric insulation (11) which is electrically insulating the outer electrode (9) of the plasma generator (6, 7), and/or
b) a gap between the outer electric insulation (11) and the housing (2) for allowing a gas flow through the gap.

5. Treating device (1) according to one of claims 3 to 4, wherein the plasma generator (6, 7) is arranged within the housing (2).

6. Treating device (1) according to any of claims 2 to 5, further comprising a radiation shielding (12) being arranged between the plasma generator (6, 7) and the object within the housing (2) thereby shielding the object against ultraviolet radiation generated by the plasma generator (6, 7).

7. Treating device (1) according to claim 6, wherein the radiation shielding (12) is gas permeable so that the plasma can flow through the radiation shielding (12) and reach the object.

8. Treating device (1), wherein
a) the radiation shielding (12) comprises several spaced apart shielding elements (15, 16), and/or
b) the shielding elements (15, 16) are curved or angled so that there is no intervisibility between the opposing sides of the radiation shielding (12).

9. Treating device (1) according to claim 8, wherein
a) the shielding elements (15, 16) are lamellas and
b) the lamellas (15, 16) are arranged in at least two adjacent layers (13, 14), and
c) the lamellas (15, 16) in the adjacent layers (13, 14) are oppositionally angled.

10. Treating device (1) according to claim 8 or 9, wherein
a) the radiation shielding (12) and/or the shielding elements (15, 16) consist of or are coated with an electrically conductive material, and/or
b) the electrically conductive material is metal, particularly copper or tin, and/or
c) the radiation shielding (12) and/or the shielding elements (15, 16) are electrically grounded.

11. Treating device (1) according to any of claims 3 to 10, wherein
a) the electrodes (9, 10) and/or the barrier (8) and/or the outer insulation (11) and/or the radiation shielding (12) is substantially flat and/or layer-shaped, and/or
b) the electrodes (9, 10) and/or the barrier (8) and/or the outer insulation (11) comprise a wire mesh.

12. Treating device (1) according to any of the preceding claims, further comprising a spacer for preventing a physical contact between the plasma generator (6, 7) and the object within the housing (2).

13. Treating device (1) according to claim 12, wherein
a) the spacer is substantially flat and/or
b) the spacer comprises a wire mesh.

14. Treating device (1) according to any of the preceding claims, wherein
a) the housing (2) comprises an outer wall consisting of an electrically conductive material, and/or
b) the outer wall of the housing (2) is electrically grounded.

15. Treating device (1) according to any of the preceding claims, wherein
a) the inlet opening (3) in the housing (2) is suitable for introducing a hand of a human being through the inlet opening (3) into the housing (2), and/or
b) the inlet opening (3) in the housing (2) comprises a height which is greater than 2cm or 3cm and/or smaller than 20cm, 10cm, 7cm or 5cm, and/or
c) the inlet opening (3) in the housing (2) comprises a width, which is greater than 5cm, 7cm or 9cm and/or smaller than 30cm, 20cm, 15cm, 13cm or 11cm, and/or
d) the housing (2) is sufficiently large for introducing a hand of a human being into the housing (2), and/or
e) the housing (2) comprises an inner length, which is greater than 5cm, 7cm or 10cm and/or smaller than 30cm, 20cm, 15cm, 13cm or 12cm, and/or
f) the housing (2) comprises an inner width, which is greater than 5cm, 7cm or 10cm and/or smaller than 30cm 20cm, 15cm or 12cm, and/or
g) the housing (2) comprises an inner height, which is greater than 4cm, 5cm or 6cm and/or smaller than 20cm, 15cm, 10cm or 8cm.

16. Use of the treating device (1) according to any of the preceding claims for applying a non-thermal plasma to an object of treatment, wherein the object of treatment is selected from a group consisting of:
a) an extremity of a human being, particularly a hand or a foot,
b) a surgical instrument,
c) an implant, particularly a heart pacemaker, a stent, an artificial joint,
d) other devices to be sterilised.
